(19) Europäisches Patentamt
European Patent Office
Office européen des brevets

(11) **EP 3 283 454 B1**

(12) **FASCICULE DE BREVET EUROPEEN**

(45) Date de publication et mention de la délivrance du brevet:
**20.03.2019 Bulletin 2019/12**

(21) Numéro de dépôt: **16719237.6**

(22) Date de dépôt: **11.04.2016**

(51) Int Cl.:
**C07C 17/386** *(2006.01)*    **C07C 19/08** *(2006.01)*

(86) Numéro de dépôt international:
**PCT/EP2016/057882**

(87) Numéro de publication internationale:
**WO 2016/166045 (20.10.2016 Gazette 2016/42)**

(54) **PROCÉDÉ DE PURIFICATION DU PENTAFLUOROÉTHANE**

VERFAHREN ZUR REINIGUNG VON PENTAFLUOROETHAN

METHOD FOR PURIFYING PENTAFLUOROETHANE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.04.2015 FR 1553226**

(43) Date de publication de la demande:
**21.02.2018 Bulletin 2018/08**

(73) Titulaire: **Arkema France**
**92700 Colombes (FR)**

(72) Inventeurs:
• **ANDRE, David**
**69530 Brignais (FR)**
• **BABA-AHMED, Abdelatif**
**69190 Saint-Fons (FR)**

(74) Mandataire: **Hirsch & Associés**
**137, rue de l'Université**
**75007 Paris (FR)**

(56) Documents cités:
WO-A1-95/21148        WO-A1-99/44973
WO-A1-2012/011609     WO-A1-2012/079011
FR-A1- 2 215 407      FR-A1- 2 758 137
US-A- 3 113 079       US-A- 3 689 372
US-A- 5 207 876       US-A1- 2011 270 001

• **LOU ET AL: "Separation of HFC-125 and CFC-115", JINGXI HUAGONG - FINE CHEMICALS, ZHONGGUO HUAGONG XUEHUI, DALIAN, CN, vol. 23, no. 8, 1 août 2006 (2006-08-01), pages 762-764, XP008178826, ISSN: 1003-5214**

## Description

DOMAINE DE L'INVENTION

L'invention concerne la purification du pentafluoroéthane (R125) contenant du chloropentafluoroéthane (R115) et a plus particulièrement pour objet un procédé de purification dans lequel le R115 est éliminé par distillation extractive.

ARRIERE-PLAN TECHNIQUE

Le pentafluoroéthane est l'un des substituts possibles des chlorofluorocarbures (CFC) qui sont concernés par le protocole de Montréal et qui se caractérisent par une durée de vie exceptionnellement élevée leur permettant d'atteindre les hautes couches de l'atmosphère et de participer ainsi sous l'influence du rayonnement UV à la destruction de la couche d'ozone. Il est donc évident que leurs substituts ne devront, en fonction des divers procédés d'obtention, ne renfermer que des traces de ces CFC.

Les substituts sont généralement obtenus soit par des méthodes de fluoruration appropriées qui ne sont pas hautement sélectives et peuvent générer par dismutation des composés perhalogénés du type CFC, soit à partir de CFC eux-mêmes par des méthodes de réduction, en pratique par des réactions d'hydrogénolyse. C'est ainsi que le pentafluoroéthane (R125) peut être préparé par fluoration du perchloroéthylène ou de ses produits de fluoration intermédiaires tels que le dichlorotrifluoroéthane (R123) et le chlorotétrafluoroéthane (R124), ou par hydrogénolyse du chloropentafluoroéthane (R115). Dans les deux cas, le R125 produit contient des quantités non négligeables de R115 qu'il convient, d'éliminer le plus complètement possible, le R115 étant un CFC.

Or, l'existence d'un azéotrope R115/R125 à 21 % en poids de R115 (voir le brevet US 3,505,233) avec un point d'ébullition (-48,5°C sous 1,013 bar) très voisin de celui du R125 (-48,1°C) rend pratiquement impossible la séparation complète du R115 et du R125 par distillation à moins d'utiliser des procédés techniquement complexes tels que la distillation azéotropique à différentes pressions comme décrit dans le brevet US 5,346,595. L'élimination du R115 dans le R125 ne peut donc se faire que par voie chimique ou par des méthodes physiques mettant en jeu un tiers corps.

Dans la demande de brevet EP 0 508 631 qui décrit la production d'hydrofluorocarbures (HFC) par réduction chimique en phase liquide de composés chlorés, bromés ou iodés avec un hydrure métallique ou un complexe d'un tel hydrure, il est indiqué que ce procédé peut être intéressant pour purifier certains HFC comme le pentafluoroéthane. Dans le même but, la demande de brevet japonais publiée (Kokai) sous le n°2001414/90 utilise des couples rédox métalliques en milieu solvant. D'autres techniques comme celle décrite dans Journal of Fluorine Chemistry, 1991 vol. 55, p.105-107, utilisent des réducteurs organiques tels que le formiate d'ammonium en milieu DMF et en présence de persulfate d'ammonium.

Ces procédés qui font appel à des réactifs difficiles à manipuler (hydrures métalliques) ou susceptibles de poser des problèmes d'effluents, sont peu compatibles avec une production industrielle de R125 en tonnages importants.

Pour une fabrication industrielle de R125, la technique de distillation extractive apparaît être le procédé idéal pour éliminer le R115 résiduel.

Dans un procédé de distillation extractive, la séparation des constituants d'un mélange binaire se fait à l'aide d'une colonne dite d'extraction comportant successivement, du bouilleur à la tête, trois tronçons, l'un d'épuisement, le second d'absorption et le troisième de récupération.

Le mélange binaire à fractionner est injecté en tête du tronçon d'épuisement alors que le tiers corps jouant le rôle de solvant sélectif ou agent d'extraction est introduit en tête du tronçon d'absorption de manière à circuler à l'état liquide de son point d'introduction jusqu'au bouilleur.

Le troisième tronçon dit de récupération sert à séparer par distillation le constituant le moins absorbé, des traces de solvant entraînées sous l'effet de sa tension de vapeur non nulle.

Le document EP 0 669 302 décrit un procédé de purification du pentafluoroéthane contenant du chloropentafluoroéthane par distillation extractive, l'agent extractant étant un alcane ou un cycloalcane en C5 à C8.

Le document FR 2 758 137 décrit un procédé de purification du pentafluoroéthane contenant du chloropentafluoroéthane par distillation extractive, l'agent extractant étant un halogénure de perfluoroalkyle en C5 à C8.

Le document FR 2 730 228 décrit un procédé de purification du pentafluoroéthane contenant du chloropentafluoroéthane par distillation extractive, l'agent extractant étant le perchloroéthylène.

Le document WO 96/06063 décrit un procédé de purification du pentafluoroéthane comprenant la mise en contact du pentafluoroéthane avec un composé organique polaire liquide comprenant au moins un atome d'azote et/ou un atome d'oxygène.

WO 95/21148 décrit un procédé d'extraction distillative utilisant les alcools (méthanol, éthanol).

Lou, dans Jingxi Huagong fine chemicals zhongguo huagong xuehui, dalian, vol.23, numéro 8, pages 762-764. décrit l'utlisation du cyclohexane, l'acétonitrile, le nitrométhane, les mélanges acétone-cyclohexane, mélange acétate d'éthyle- acétate de butyle, et les mélanges alcool industriel et acétate d'éthyle dans un procédé de distillation extractive afin de purifier le pentafluoroéthane.

Certains de ces agents d'extraction ne satisfont pas les conditions imposées en termes d'exigences environnementales.

Il a été trouvé des agents d'extraction permettant de satisfaire les exigences environnementales et présentant une sélectivité et/ou une capacité améliorée.

RESUME DE L'INVENTION

L'invention concerne en premier lieu un procédé de purification du pentafluoroéthane contenant du chloropentafluoroéthane par distillation extractive, ledit procédé comprenant l'utilisation d'un agent d'extraction choisi parmi le diméthylformamide, le dioxane, le diméthylsulfoxyde et le diéthylsulfoxyde.

Selon un mode de réalisation particulier, l'agent d'extraction est choisi parmi le diméthylsulfoxyde et le diéthylsulfoxyde.

De préférence, la distillation est effectuée sous une pression allant de la pression atmosphérique à 20 bars.

Selon un mode de réalisation de l'invention, le ratio molaire pentafluoroéthane/chloropentafluoroéthane avant distillation va de 2 à 99.

Selon un mode de réalisation de l'invention, le ratio molaire pentafluoroéthane/chloropentafluoroéthane après distillation va de 5 à 99999.

Selon un mode de réalisation de l'invention, le mélange contenant le pentafluoroéthane et le chloropentafluoroéthane à purifier est issu de la réaction de fluoration du perchloroéthylène ou d'un produit de fluoration intermédiaire dudit perchloroéthylène.

Selon un mode de réalisation de l'invention, le produit de fluoration intermédiaire dudit perchloroéthylène est choisi parmi le dichlorotrifluoroéthane ou le chlorotétrafluoroéthane.

Selon un mode de réalisation de l'invention, le mélange contenant le pentafluoroéthane et le chloropentafluoroéthane à purifier est issu de la réaction d'hydrogénolyse du chloropentafluoroéthane.

Le procédé selon l'invention est simple à mettre en oeuvre, en particulier à l'échelle industrielle.

Le procédé selon l'invention permet de récupérer le pentafluoroéthane avec une plus grande pureté.

Le procédé selon l'invention utilise des solvants ayant une plus faible toxicité et présentant ainsi un plus faible impact sur l'environnement.

Le procédé selon l'invention présente une sélectivité améliorée et/ou une capacité améliorée.

DESCRIPTION DE MODES DE REALISATION DE L'INVENTION

L'invention est maintenant décrite plus en détail et de façon non limitative dans la description qui suit.

La présente invention propose un procédé de purification d'un pentafluoroéthane (R125) contenant du chloropentafluoroéthane (R115) par distillation extractive, ledit procédé comprenant l'utilisation d'un agent d'extraction choisi parmi le diméthylformamide (DMF), le dioxane, le diméthylsulfoxyde (DMSO) et le diéthylsulfoxyde (DESO).

Le dioxane peut être le 1,4-dioxane, le 1,3-dioxane ou le 1,2-dioxane. De préférence, le dioxane est le 1,4-dioxane.

Selon un mode de réalisation de l'invention, l'agent d'extraction est choisi parmi le dioxane, le diméthylsulfoxyde et le diéthylsulfoxyde.

Selon un mode de réalisation de l'invention, l'agent d'extraction est choisi parmi le diméthylsulfoxyde et le diéthylsulfoxyde.

Selon un mode de réalisation de l'invention, le pentafluoroéthane à purifier comprend notamment du pentafluoroéthane et du chloropentafluoroéthane.

Selon un mode de réalisation, le ratio molaire R125/R115 avant distillation va de 2 à 99.

Selon un mode de réalisation, le ratio molaire R125/R115 après distillation va de 5 à 99999.

Selon un mode de réalisation, le pentafluoroéthane purifié comprend moins de 10 ppm massique de R115.

De préférence, le procédé selon l'invention est mis en oeuvre sans la présence d'hydrures métalliques ou de sels d'ammonium.

Le procédé selon l'invention peut être mis en oeuvre selon les principes bien connus de la distillation extractive. L'opération peut être effectuée dans une colonne de distillation extractive dans laquelle le mélange R125-R115 à séparer est injecté en un point situé en tête du tronçon d'épuisement. L'agent d'extraction, est introduit dans la colonne en un point situé en tête du tronçon d'absorption; il circule à l'état liquide de son point d'introduction jusqu'au bouilleur.

Le diamètre et le nombre d'étages de la colonne de distillation extractive, le taux de reflux et les températures et pressions optimales peuvent être facilement calculées par l'homme du métier à partir des données propres aux constituants individuels et à leurs mélanges (volatilités relatives, pressions de vapeur et constantes physiques).

Selon un mode de réalisation de l'invention, la distillation est effectuée sous une pression allant de la pression atmosphérique à 20 bars absolus.

Selon un mode de réalisation de l'invention, la distillation est effectuée à une température allant de -50°C à 250°C, de préférence de -10°C à 200°C, de préférence encore de 5°C à 150°C.

Lors du procédé de purification selon l'invention, l'agent d'extraction absorbe sélectivement le R125.

Dans ce mode de réalisation, l'agent d'extraction présente alors de préférence un facteur de séparation F à 25°C, tel que défini ci-dessous, supérieur à 2, de préférence supérieur à 3, de préférence encore supérieur à 4.

Le facteur de séparation (F) est défini de la façon suivante :

$$F = \frac{\gamma(\text{R115})}{\gamma(\text{R125})} \times \frac{P(\text{R115})}{P(\text{R125})}$$

où $\gamma(\text{R125})$ représente le coefficient d'activité du composé R125 dans le solvant considéré à dilution infinie.

$\gamma(\text{R115})$ représente le coefficient d'activité du composé R115 dans le solvant considéré à dilution infinie.

P(R125) représente la tension de vapeur du composé R125 à la température considérée.

P(R115) représente la tension de vapeur du composé R115 à la température considérée.

Le ratio P(R125)/P(R115) correspond à la volatilité relative du R125 par rapport au R115.

Et comme dans le cas précis de la présente invention, le rapport des tensions de vapeur est constant quel que soit le solvant d'extraction utilisé, nous pouvons considérer un facteur de séparation simplifié F' ou sélectivité S défini par :

$$F' = \frac{\gamma(\text{R115})}{\gamma(\text{R125})}$$

Les valeurs de coefficients d'activité des composés i (i est R125 ou R115), $\gamma_i$, sont calculées selon la relation :

$\ln \gamma_j = (\mu_j^i - \mu_j^p)/RT$, où $\mu_j^i$ correspond au potentiel chimique du composé i à dilution infinie dans le solvant considéré, et $\mu_j^p$ correspond au potentiel chimique du composé pur i, et R est la constante des gaz parfaits, et T est la température.

Le coefficient d'activité et la tension de vapeur sont des données bien connues et accessibles à l'homme du métier.

La capacité d'un solvant est définie par l'inverse du coefficient d'activité du soluté dans le solvant à dilution infinie.

Selon un mode de réalisation de l'invention, le procédé de purification selon l'invention comprend en outre une étape ultérieure de séparation du solvant d'extraction et du composé R125 comme cela est connu de l'homme de l'art et décrit par exemple dans le document EP 778254. Une colonne de régénération du solvant permet de séparer le mélange solvant/R125 selon leur différence de point d'ébullition. Le solvant récupéré peut être réutilisé pour la distillation extractive.

La sélectivité et la capacité de différents solvants sont indiquées dans le tableau 1 suivant :

Tableau 1 : capacité et sélectivité des solvants

| Solvant | Facteur de Séparation simplifié (F') = sélectivité S | Capacité |
|---|---|---|
| Acétonitrile | 6,31 | 0,3 |
| THF | 7,64 | 2,09 |
| Hexane | 0,63 | 0,18 |
| Acétone | 9,86 | 1,29 |
| DMF | 16,05 | 1,63 |
| 1,4-dioxane | 8,86 | 1,12 |
| diméthylsulfoxyde | 30,08 | 0,73 |
| Diéthylsulfoxyde | 22,72 | 1,94 |

Les données du tableau 1 montrent qu'à l'exception de l'hexane, tous ces solvants présentent de bonnes caractéristiques d'extraction.

Il est montré ici que de nouveaux solvants tels que le DMF, le 1,4-dioxane, le DMSO et le DESO donnent de très bons résultats en termes de sélectivité et/ou de capacité. Ces 4 solvants présentent un excellent choix en termes de performances pour la distillation extractive. Par ailleurs, ces solvants ne sont pas, ou peu, nocifs pour l'environnement.

En particulier, le DMSO et le DESO présentent d'excellentes sélectivités, respectivement de 30,08 et 22,72.

Au contraire, d'autres solvants traditionnellement utilisés, tels que l'acétonitrile, ne présentent pas une bonne sélectivité et une bonne capacité.

## Revendications

1. Procédé de purification du pentafluoroéthane contenant du chloropentafluoroéthane par distillation extractive, ledit procédé comprenant l'utilisation d'un agent d'extraction choisi parmi le diméthylformamide, le dioxane, le diméthylsulfoxyde et le diéthylsulfoxyde.

2. Procédé selon la revendication 1, dans lequel l'agent d'extraction est choisi parmi le diméthylsulfoxyde et le diéthylsulfoxyde.

3. Procédé selon la revendication 1 ou 2, dans lequel la distillation est effectuée sous une pression allant de la pression atmosphérique à 20 bars.

4. Procédé selon l'une quelconque des revendications 1 à 3, dans lequel le ratio molaire pentafluoroéthane/chloro-pentafluoroéthane avant distillation va de 2 à 99.

5. Procédé selon l'une quelconque des revendications 1 à 4, dans lequel le ratio molaire pentafluoroéthane/chloro-pentafluoroéthane après distillation va de 5 à 99999.

6. Procédé selon l'une quelconque des revendications 1 à 5, dans lequel le mélange contenant le pentafluoroéthane et le chloropentafluoroéthane à purifier est issu de la réaction de fluoration du perchloroéthylène ou d'un produit de fluoration intermédiaire dudit perchloroéthylène.

7. Procédé selon la revendication 6, dans lequel le produit de fluoration intermédiaire dudit perchloroéthylène est choisi parmi le dichlorotrifluoroéthane ou le chlorotétrafluoroéthane.

8. Procédé selon l'une quelconque des revendications 1 à 5, dans lequel le mélange contenant le pentafluoroéthane et le chloropentafluoroéthane à purifier est issu de la réaction d'hydrogénolyse du chloropentafluoroéthane.

## Patentansprüche

1. Verfahren zur Reinigung von Pentafluorethan, das Chlorpentafluorethan enthält, durch Extraktivdestillation, wobei das Verfahren die Verwendung eines Extraktionsmittels umfasst, ausgewählt aus Dimethylformamid, Dioxan, Dimethylsulfoxid und Diethylsulfoxid.

2. Verfahren nach Anspruch 1, wobei das Extraktionsmittel ausgewählt ist aus Dimethylsulfoxid und Diethylsulfoxid.

3. Verfahren nach Anspruch 1 oder 2, wobei die Destillation bei einem Druck im Bereich von Atmosphärendruck bis 20 bar durchgeführt wird.

4. Verfahren nach einem der Ansprüche 1 bis 3, wobei das Pentafluorethan/Chlorpentafluorethan-Molverhältnis vor der Destillation im Bereich von 2 bis 99 liegt.

5. Verfahren nach einem der Ansprüche 1 bis 4, wobei das Pentafluorethan/Chlorpentafluorethan-Molverhältnis nach der Destillation im Bereich von 5 bis 99.999 liegt.

6. Verfahren nach einem der Ansprüche 1 bis 5, wobei die Mischung, enthaltend das Pentafluorethan und das zu reinigende Chlorpentafluorethan, aus der Fluorierungsreaktion von Perchlorethylen oder aus einem Fluorierungszwischenprodukt des Perchlorethylens stammt.

7. Verfahren nach Anspruch 6, wobei das Fluorierungszwischenprodukt des Perchlorethylens ausgewählt ist aus Dichlortrifluorethan oder Chlortetrafluorethan.

8. Verfahren nach einem der Ansprüche 1 bis 5, wobei die Mischung, enthaltend das Pentafluorethan und das zu reinigende Chlorpentafluorethan, aus der Hydrogenolysereaktion von Chlorpentafluorethan stammt.

**Claims**

1. Process for the purification of pentafluoroethane containing chloropentafluoroethane by extractive distillation, said process comprising the use of an extractant selected from dimethylformamide, dioxane, dimethylsulphoxide and diethylsulphoxide.

2. Process according to claim 1, in which the extractant is selected from dimethylsulphoxide and diethylsulphoxide.

3. Process according to claim 1 or 2, in which the distillation is carried out under a pressure ranging from atmospheric pressure to 20 bars.

4. Process according to any one of claims 1 to 3, in which the pentafluoroethane/chloropentafluoroethane molar ratio before distillation ranges from 2 to 99.

5. Process according to any one of claims 1 to 4, in which the pentafluoroethane/chloropentafluoroethane molar ratio after distillation ranges from 5 to 99999.

6. Process according to any one of claims 1 to 5, in which the mixture containing pentafluoroethane and chloropentafluoroethane to be purified originates from the fluorination reaction of perchloroethylene or from an intermediate fluorination product of said perchloroethylene.

7. Process according to claim 6, in which the intermediate fluorination product of said perchloroethylene is selected from dichlorotrifluoroethane or chlorotetrafluoroethane.

8. Process according to any one of claims 1 to 5, in which the mixture containing pentafluoroethane and chloropentafluoroethane to be purified originates from the hydrogenolysis reaction of chloropentafluoroethane.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3505233 A
- US 5346595 A
- EP 0508631 A
- JP 200141490 B
- EP 0669302 A
- FR 2758137
- FR 2730228
- WO 9606063 A
- WO 9521148 A
- EP 778254 A

**Littérature non-brevet citée dans la description**

- *Journal of Fluorine Chemistry,* 1991, vol. 55, 105-107
- **LOU.** *Jingxi Huagong fine chemicals zhongguo huagong xuehui,* vol. 23 (8), 762-764